# EUROPÄISCHE PATENTSCHRIFT

(11) **EP 1 737 896 B1**
(45) Veröffentlichungstag und Bekanntmachung des Hinweises auf die Patenterteilung: **05.11.2008**
(21) Anmeldenummer: 05744213.9
(22) Anmeldetag: 22.04.2005
(51) Int. Cl.: C08F 18/08, C08F 16/06

(54) **NEUE HYDROGELE AUF BASIS VON POLYVINYLALKOHOLEN UND POLYVINYLALKOHOL-COPOLYMEREN**
NOVEL HYDROGELS BASED ON POLYVINYL ALCOHOLS AND POLYVINYL ALCOHOL COPOLYMERS
NOUVEAUX HYDROGELS A BASE D'ALCOOLS POLYVINYLIQUES ET DE COPOLYMERES D'ALCOOLS POLYVINYLIQUES

(30) Priorität: 22.04.2004 DE 102004019504
(43) Veröffentlichungstag der Anmeldung: 03.01.2007
(73) Patentinhaber: Celanese Ventures GmbH, 65926 Frankfurt am Main (DE)
(72) Erfinder: SCHULTE, Jörg, 60487 Frankfurt am Main (DE); DICKNER, Tim, 60486 Frankfurt am Main (DE); BRUCKMANN, Monika, 45144 Essen (DE); SCHOTTEK, Jörg, 65510 Idstein/Wörsdorf (DE); BLANK, Uwe, 65187 Wiesbaden (DE)
(74) Vertreter: Luderschmidt, Schüler & Partner
(86) Internationale Anmeldenummer: PCT/EP2005/004348
(87) Internationale Veröffentlichungsnummer: WO 2005/103097

(56) Entgegenhaltungen:
- WO-A-20/04018532
- US-A1- 2002 151 650
- GRIFFITH CIMA L. AND S.T. LOPINA: "network structures of radiation crosslinked star polymer gels" MACROMOLECULES, Bd. 28, 1995, Seiten 6787-6794, XP002337097

## Beschreibung

Die vorliegende Erfindung betrifft Hydrogele enthaltend Polyvinylalkohole und/oder Polyvinylalkohol-Copolymere, Verfahren zur Herstellung der Hydrogele, und die Verwendung der Hydrogele als Materialien im biomedizinischen und pharmazeutischen Bereich, zur Herstellung von Kontaktlinsen, zur kontrollierten Freisetzung von Wirkstoffen, als Trägermaterial für Einschluß - immobilisierte Biokatalysatoren, als Trägermaterial für Übergangsmetallkatalysatoren, als Material für die Herstellung von Reaktivmembranen, als Zusatz zu Bohrflüssigkeiten, als Verdrängungsmittel bei der Ölförderung, als Additiv für Zement und als Zusatzmittel zu hochviskosen Flüssigkeiten, wie z.B. Erdöl zur Verbesserung des Fließverhaltens und zum schnelleren Transport, sowie als Bestandteil von Kosmetikartikeln.

Hydrogele sind Wasser enthaltende Gele auf der Basis hydrophiler Polymere, die als dreidimensionale Netzwerke vorliegen. Als Hydrogele sind diese Polymere in Wasser unlöslich, quellen aber unter weitgehender Formerhaltung bis zu einem Gleichgewichtsvolumen auf. Die Netzwerkbildung erfolgt vorwiegend über chemische Verknüpfung der einzelnen Polymerketten, ist aber auch physikalisch durch elektrostatische, hydrophobe oder Dipol/Dipol-Wechselwirkungen zwischen einzelnen Segmenten der Polymerketten möglich. Über die Wahl der zum Polymeraufbau verwendeten Monomeren, die Art der Vernetzung und die Vernetzungsdichte können gewünschte Eigenschaften der Hydrogele gezielt eingestellt werden. Die notwendige Hydrophilie der Polymeren vermitteln u.a. Hydroxy -, Carboxylat -, Sulfonat - oder Amid - Gruppen. Synthetische Hydrogele basieren u.a. auf Poly(meth)acrylsäuren, Poly(meth)acrylaten, Polyvinylpyrrolidon oder Polyvinylalkohol. Hydrogele sind im allgemein gut verträglich mit lebenden Geweben (Quelle: Römpp Lexikon Chemie - Version 2.0, Stuttgart/New York: Georg Thieme Verlag 1999).

Hydrogele auf der Basis von Polyvinylalkohol sind bekannt (vgl. US2003008396A1, US2001029399A1, US0006231605B1, US0005981826A, WO2001044307A3, WO1998050017A1 Hassan, Christie M.; Peppas, Nikolaos, A.; Advances in Polymer Science (2000), 153 (Biopolymers, PVA Hydrogels Anionic Polymerisation Nanocomposites), 37-65., Peppas, Nikolaos A. , Hydrogels Med. Pharm. (1987), 2, 1-48. und dort zitierte Literatur.). Hydrogele auf der Basis von Polyvinylalkohol können durch unterschiedliche Methoden hergestellt werden, wie beispielsweise durch wiederholtes Einfrieren und Auftauen einer wäßrigen Lösung von Polyvinylalkohol, durch Einwirkung ionisierender Strahlung (UV-Licht, gamma-Strahlung) auf eine wäßrige Lösung von Polyvinylalkohol, und durch Reaktion von Polyvinylalkohol mit vernetzenden Reagenzien wie Glutaraldehyd, Acetaldehyd, Formaldehyd, Maleinsäure, Oxalsäure, Alginsäure, Dimethylharnstoff, Glyoxal, Salzsäure, Polyacrolein, Diisocyanaten und/oder Divinylsulfat.

Hydrogele auf der Basis von Polyvinylalkoholen werden in der Regel aus konventionellem, linearen Polyvinylalkohol hergestellt. Hierbei tritt eine Trübung des Hydrogels durch kristalline Bereiche auf, welche dazu führt, daß die Hydrogele aus konventionellem Polyvinylalkohol nur bedingt für optische Anwendungen, wie beispielsweise Kontaktlinsen, geeignet sind. Desweiteren erhalten Hydrogele aus konventionellem Polyvinylalkohol ihre mechanischen Eigenschaften erst bei der Vernetzung zum Hydrogel.

Überraschenderweise wurde nun gefunden, daß Hydrogele aus Polyvinylalkoholen mit speziellen Geometrien, wie beispielsweise sternförmigen Geometrien, wie sie in DE10343607 (Schulte et al. / Celanese Ventures GmbH) und DE10356574 (Bruckmann et al. / Celanese Ventures GmbH) beschrieben sind, einen geringeren Mikrokristallinitätsgrad im Hydrogel hervorrufen, was zu verbesserten optischen Eigenschaften führt, und verbesserte mechanische Eigenschaften aufweisen. Desweiteren führt die spezielle Geometrie der Polyvinylalkohole durch eine Vernetzung der Polymere vor der Hydrogel-Bildung zu einem verringerten Abrieb.

Gegenstand der vorliegenden Erfindung sind somit Hydrogele enthaltend mindestens ein Polyvinylalkohol-Sternpolymer und weitere Komponenten.

Im Rahmen der vorliegenden Erfindung werden unter Hydrogelen enthaltend mindestens ein Polyvinylalkohol-Sternpolymer sowohl Hydrogele, bei denen als Polymer exklusiv ein oder mehrere Polyvinylalkohol-Sternpolymere eingesetzt werden, als auch Mischungen zwischen Polyvinylalkohol-Sternpolymeren mit anderen Polymeren verstanden. Besonders bevorzugt sind hierbei Mischungen aus Polyvinylalkohol-Sternpolymeren mit konventionellem Polyvinylalkohol im Verhältnis von 99:1 bis 1:99. Desweiteren besonders bevorzugt sind Mischungen aus Polyvinylalkohol-Sternpolymeren mit Polyacrylsäuren, Polymethacrylsäuren, Polyacrylaten, Polymethacrylaten und Polyvinylpyrrolidon im Verhältnis von 99:1 bis 1:99.

Die erfindungsgemäßen Hydrogele enthalten neben den vorstehend genannten Polyvinylalkohol-Sternpolymeren, wie der Name bereits verdeutlicht, noch Wasser.

Polyvinylalkohole mit speziellen Geometrien lassen sich über unterschiedliche Methoden herstellen, zum einen über eine Übergangsmetall-katalysierte Herstellung von Polyvinylacetat, wie er in DE10238659 beschrieben ist, gefolgt von einer Verseifung, wie er in DE10343607 beschrieben, oder über eine Copolymerisation von Vinylacetat mit vernetzenden Comonomeren, gefolgt von einer Verseifung, wie er in DE10356574 beschrieben.

Im folgenden werden die in DE10343607 und DE10356574 beschriebenen Polyvinylalkohole und Polyvinylalkohol-Copolymere als Polyvinylalkohol-Sternpolymere bezeichnet.

Unter Polyvinylalkohol-Sternpolymeren gemäß DE10343607 und DE10356574 werden Verbindungen der Formel **I**, **II** und **III** 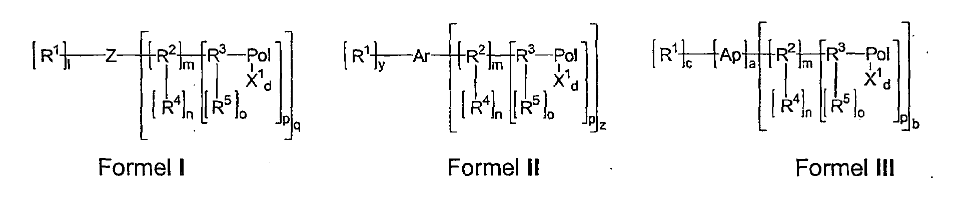 worin
- Pol: für ein Polymer auf der Basis eines Polyvinylalkohols steht, besonders bevorzugt für ein Homo- oder Copolymer auf der Basis von Polyvinylalkohol, ganz besonders bevorzugt Polyvinylalkohol, Polyvinylalkohol-Polyvinylacetat- Copolymer, Polyvinylalkohol-Polyethylen-Copolymer, Polyvinylalkohol- Polyvinylchlorid-Copolymer und Polyvinylalkohol-Polyacrylsäuremethylester- Copolymer, und
- Z: ein Zentralatom bedeutet und ein Atom der 13. bis 16. Gruppe des Periodensystems der Elemente, bevorzugt Kohlenstoff, Silicium, Stickstoff, Phosphor, Sauerstoff oder Schwefel, besonders bevorzugt Kohlenstoff oder Silicium, ist, und
- X¹: jeweils gleich oder verschieden ist, und ein Halogenatom, bevorzugt Fluor, Chlor, Brom oder lod, besonders bevorzugt Chlor, Brom oder lod ist, und
- R¹: gleich oder verschieden ist, und gleich Wasserstoff oder eine C₁ - C₂₀ - kohlenstoffhaltige Gruppe ist, und
- R²: gleich oder verschieden ist und eine verbrückende C₁ - C₂₀ -kohlenstoffhaltige Gruppe zwischen dem Zentralatom Z und der initiierenden Einheit [R³-X¹] oder Silicium oder Sauerstoff bedeutet, und
- R³: gleich oder verschieden ist und Kohlenstoff oder Silicium bedeutet, und
- R⁴: gleich oder verschieden ist und ein Wasserstoffatom oder eine C₁ - C₂₀ - kohlenstoffhaltige Gruppe ist, und
- R⁵: gleich oder verschieden ist und Wasserstoff oder eine C₁ - C₂₀ - kohlenstoffhaltige Gruppe bedeutet,
- l: eine ganze natürliche Zahl ist und für Null, 1, 2 oder 3 steht, und
- m: jeweils gleich oder verschieden ist und eine ganze natürlich Zahl ist und für Null, 1, 2, 3, 4 und 5 steht, und
- n: jeweils gleich oder verschieden ist und eine ganze natürliche Zahl ist und für Null, 1, 2, 3, 4, 5, 6, 7, 8, 9, 10, 11, 12, 13, 14, 15, 16, 17, 18, 19 und 20 steht, und
- o: jeweils gleich oder verschieden ist und 1 oder 2 ist, und
- p: jeweils gleich oder verschieden ist und eine ganze natürliche Zahl ist und für 1, 2, 3, 4 und 5 steht, und
- q: eine ganze natürliche Zahl ist und für 2, 3 und 4 steht, und
- Ar: ein aromatisches Grundgerüst mit mindestens vier Kohlenstoffatomen ist, bei dem ein oder mehrere C-Atome durch Bor, Stickstoff oder Phosphor ausgetauscht sein können, und wobei bevorzugte aromatische oder heteroaromatische Grundgerüste sich von Benzol, Biphenyl, Naphthalin, Anthracen, Phenanthren, Triphenylen, Chinolin, Pyridin, Bipyridin, Pyridazin, Pyrimidin, Pyrazin, Triazin, Benzopyrrol, Benzotriazol, Benzopyridin, Benzopyrazidin, Benzopyrimidin, Benzopyrazin, Benzotriazin, Indolizin, Chinolizin, Carbazol, Acridin, Phenazin, Benzochinolin, Phenoxazin, die gegebenenfalls auch substituiert sein können, ableiten, und
- y: eine ganze natürliche Zahl ist und für Null, 1, 2, 3, 4, 5, 6, 7, 8, 9, 10, 11, 12, 13, 14, 15, 16, 17, 18, 19 und 20 steht, und
- z: eine ganze natürliche Zahl ist und für 2, 3, 4, 5, 6, 7, 8, 9 und 10 steht.
- Ap: ein cyclisches nicht-aromatisches Grundgerüst mit mindestens drei Kohlenstoffatomen ist, welches auch Heteroatome wie Stickstoff, Bor, Phosphor, Sauerstoff oder Schwefel enthalten kann, ist, wobei sich bevorzugte aliphatische Grundgerüste aus der Gruppe Cycloalkyl, wie beispielsweise Cyclopropyl, Cyclobutyl, Cyclopentyl, Cyclohexyl, Cycloheptyl, Cyclooctyl, Cyclononyl, oder aus der Gruppe Cycloheteroalkyl, wie beispielsweise Aziridin, Azetidin, Pyrrolidin, Piperidin, Azepan, Azocan, 1,3,5-Triazinan, 1,3,5- Trioxan, Oxetan, Furan, Dihydrofuran, Tetrahydrofuran, Pyran, Dihydropyran, Tetrahydropyran, Oxepan, Oxocan, oder aus der Gruppe der Saccharide, wie beispielsweise alpha-Glucose, beta-Glucose, ableiten lassen, und
- a: eine ganze natürliche Zahl ist und für Null, 1, 2, 3, 4, 5, 6, 7, 8, 9, 10, 11, 12, 13, 14, 15, 16, 17, 18, 19 und 20 steht, und
- b: eine ganze natürliche Zahl ist und für 2, 3, 4, 5, 6, 7, 8, 9 und 10 steht, und
- c: eine ganze natürliche Zahl ist und für Null, 1, 2, 3, 4, 5, 6, 7, 8, 9, 10, 11, 12, 13, 14, 15, 16, 17, 18, 19 und 20 steht,
- d: gleich oder verschieden sein kann, und Null oder Eins ist, verstanden, sowie

Polyvinylalkohol-Copolymere auf der Basis von Polyvinylester-Copolymeren werden durch ein Verfahren umfassend die Schritte:
A) Radikalische Lösungs- oder Massen-Polymerisation von Vinylestern in Gegenwart eines Radikalbildners und gegebenenfalls in Gegenwart eines Radikalfängers,
B) Zugabe einer zur Vernetzung befähigten Polyalkenyl-Verbindung,
C) gegebenenfalls Aufarbeitung und Isolierung der gebildeten Polyvinylester-Copolymere,
D) Verseifung der in B) hergestellten Polyvinylester/Polyvinylester-polyalken-Mischung oder der unter C) isolierten Polyvinylester-polyalken-Copolymere mit Base unter Ausbildung der

Polyvinylalkohol)/Polyvinylalkohol-polyalken-Mischungen bzw.

Polyvinylalkohol-polyalken-Copolymeren und Isolierung der Produkte, erhalten, und sind dadurch gekennzeichnet, daß es sich bei den in Schritt B) eingesetzten Polyalkenyl-Verbindungen um Verbindungen gemäß Formel **IV:** 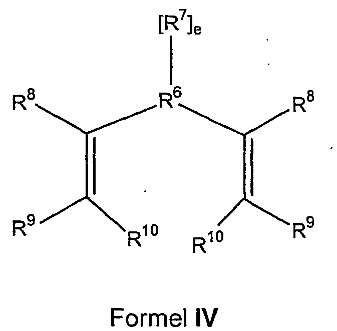 worin:
- R⁶: eine C₆ - C₂₀ -Arylgruppe, eine C₅ - C₂₀ -Heteroarylgruppe, eine C₄-C₂₀- Cycloalkylgruppe, eine C₄-C₂₀-Heterocycloalkylgruppe oder eine C₁ - C₂₀- Alkylgruppe bedeutet, bei der ein oder mehrere nicht direkt benachbarte C- Atome durch ein Element der 5. oder 6. Gruppe der Elemente, bevorzugt Stickstoff, Phosphor, Sauerstoff oder Schwefel, besonders bevorzugt Stickstoff oder Sauerstoff, ersetzt sein können, und
- R⁷: gleich oder verschieden ist, und gleich Wasserstoff, Sauerstoff, Schwefel oder eine Hydroxygruppe, eine Carbamoylgruppe, eine Aminogruppe, eine Carboxygruppe, eine C₁ - C₂₀ -Alkylcarbonylgruppe, eine C₁ - C₂₀ - Alkyloxygruppe, eine C₆ - C₂₀ -Aryloxygruppe, eine Iminogruppe, eine C₁ - C₂₀ -Alkyliminogruppe, eine C₆ - C₂₀ -Alkyliminogruppe, eine Cyanogruppe, eine C₁ - C₂₀ - Alkylgruppe, eine C₆ - C₂₀ -Arylgruppe, eine C₅ - C₂₀ - Heteroarylgruppe, eine C₄-C₂₀-Cycloalkylgruppe, eine C₄-C₂₀-Heterocycloalkyl eine C₇ - C₂₀ -Alkylarylgruppe, eine C₇ - C₃₀ -Arylalkylgruppe, eine C₂ - C₂₀ - Alkenylgruppe, eine C₂ - C₂₀ - α-Oxy-alkenyl, eine halogenhaltige C₁ - C₂₀ - Alkylgruppe, eine C₆ - C₂₀ -Arylgruppe, eine C₇ - C₂₀ -Alkylarylgruppe, eine C₇ - C₃₀ -Arylalkylgruppe oder eine C₂ - C₂₀ -Alkenylgruppe bedeutet, und
- R⁸, R⁹, R¹⁰: gleich oder verschieden sind, und gleich Wasserstoff oder eine C₁ - C₂₀ - kohlenstoffhaltige Gruppe ist, und
- e: eine ganze natürliche Zahl von 0 bis 40 bedeutet, handelt.

Im Rahmen der vorliegenden Erfindung werden unter einer C₁ - C₂₀ -kohlenstoffhaltigen Gruppe bevorzugt die Reste C₁-C₂₀-Alkyl, besonders bevorzugt Methyl, Ethyl, n-Propyl, i-Propyl, n-Butyl, i-Butyl, s-Butyl, t-Butyl, n-Pentyl, s-Pentyl, Cyclopentyl, n-Hexyl, Cyclohexyl, n-Octyl oder Cyclooctyl, C₁ - C₂₀ - Alkenyl, besonders bevorzugt Ethenyl, Propenyl, Butenyl, Pentenyl, Cyclopentenyl, Hexenyl, Cyclohexenyl, Octenyl oder Cyclooctenyl, C₁ - C₂₀ - Alkinyl, besonders bevorzugt Ethinyl, Propinyl, Butinyl, Pentinyl, Hexinyl oder Octinyl, C₆-C₂₀-Aryl, besonders bevorzugt Phenyl, Biphenyl, Naphthyl oder Anthracenyl, C₁ - C₂₀ - Fluoralkyl, besonders bevorzugt Trifluormethyl, Pentafluorethyl oder 2,2,2-Trifluorethyl, C₆-C₂₀-Aryl, besonders bevorzugt Phenyl, Biphenyl, Naphthyl, Anthracenyl, Triphenylenyl, [1,1';3',1"]Terphenyl-2'-yl, Binaphthyl oder Phenanthrenyl, C₆-C₂₀-Fluoraryl, besonders bevorzugt Tetrafluorophenyl oder Heptafluoronaphthyl, C₁-C₂₀-Alkoxy, besonders bevorzugt Methoxy, Ethoxy, n-Propoxy, i-Propoxy, n-Butoxy, i-Butoxy, s-Butoxy oder t-Butoxy, C₆-C₂₀-Aryloxy, besonders bevorzugt Phenoxy, Naphthoxy, Biphenyloxy, Anthracenyloxy, Phenanthrenyloxy, C₇-C₂₀-Arylalkyl, besonders bevorzugt o-Tolyl, m-Tolyl, p-Tolyl, 2,6-Dimethylphenyl, 2,6-Diethylphenyl, 2,6-Di-i-propylphenyl, 2,6-Di-t-butylphenyl, o-t-Butylphenyl, m-t-Butylphenyl, p-t-Butylphenyl, C₇-C₂₀-Alkylaryl, besonders bevorzugt Benzyl, Ethylphenyl, Propylphenyl, Diphenylmethyl, Triphenylmethyl oder Naphthalinylmethyl, C₇-C₂₀-Aryloxyalkyl, besonders bevorzugt o-Methoxyphenyl, m-Phenoxymethyl, p-Phenoxymethyl, C₁₂-C₂₀-Aryloxyaryl, besonders bevorzugt p-Phenoxyphenyl, C₅-C₂₀-Heteroaryl, besonders bevorzugt 2-Pyridyl, 3-Pyridyl, 4-Pyridyl, Chinolinyl, Isochinolinyl, Acridinyl, Benzochinolinyl oder Benzoisochinolinyl, C₄-C₂₀-Heterocycloalkyl, besonders bevorzugt Furyl, Benzofuryl, 2-Pyrolidinyl, 2-Indolyl, 3-Indolyl, 2,3-Dihydroindolyl, C₈-C₂₀-Arylalkenyl, besonders bevorzugt o-Vinylphenyl, m-Vinylphenyl, p-Vinylphenyl, C₈-C₂₀-Arylalkinyl, besonders bevorzugt o-Ethinylphenyl, m-Ethinylphenyl oder p-Ethinylphenyl, C₂ - C₂₀ - heteroatomhaltige Gruppe, besonders bevorzugt Carbonyl, Benzoyl, Oxybenzoyl, Benzoyloxy, Acetyl, Acetoxy oder Nitril verstanden, wobei eine oder mehrere C₁-C₂₀-kohlenstoffhaltige Gruppen ein cyclisches System bilden können.

Im Rahmen der vorliegenden Erfindung werden unter einer verbrückenden C₁ - C₂₀ - kohlenstoffhaltigen Gruppe bevorzugt C₁-C₂₀-Alkyl, besonders bevorzugt Methylen, Ethylen, Propylen, Butylen, Pentylen, Cyclopentylen, Hexylen oder Cyclohexylen, C₁-C₂₀-Alkenyl, besonders bevorzugt Ethenyl, Propenyl, Butenyl, Pentenyl, Cyclopentenyl, Hexenyl oder Cyclohexenyl, C₁-C₂₀-Alkinyl, besonders bevorzugt Ethinyl, Propinyl, Butinyl, Pentinyl oder Hexinyl, C₁-C₂₀-Aryl, besonders bevorzugt o-Phenylen, m-Phenylen oder p-Phenylen, C₁ - C₂₀ - heteroatomhaltige Gruppe, besonders bevorzugt Carbonyl, Oxycarbonyl, Carbonyloxy, Carbamoyl oder Amido verstanden.

Weiterer Gegenstand der vorliegenden Erfindung ist die Herstellung und die Verwendung der erfindungsgemäßen Hydrogele.

Die Herstellung der erfindungsgemäßen Hydrogele enthaltend ein Polyvinylalkohol-Sternpolymer und weitere Komponenten kann beispielsweise durch:
- wiederholtes Einfrieren und Auftauen einer wäßrigen Lösung enthaltend ein Polyvinylalkohol-Sternpolymer und weitere Komponenten,
- Einwirkung ionisierender Strahlung (UV-Licht, gamma-Strahlung) auf eine wäßrige Lösung enthaltend ein Polyvinylalkohol-Sternpolymer und weitere Komponenten und
- Reaktion in wäßriger Lösung enthaltend ein Polyvinylalkohol-Sternpolymer und weitere Komponenten mit vernetzenden Reagenzien wie beispielsweise Glutaraldehyd, Acetaldehyd, Formaldehyd, Maleinsäure, Oxalsäure, Alginsäure, Dimethylharnstoff, Glyoxal, Salzsäure, Polyacrolein, Diisocyanaten und/oder Divinylsulfat, gegebenenfalls unter Säure- oder Base-Katalyse,
erfolgen.

Unter weiteren Komponenten werden unter anderem:
- konventioneller Polyvinylalkohol,
- andere Polymere wie beispielsweise Polyacrylsäuren, Polymethacrylsäuren, Polyacrylaten, Polymethacrylaten und Polyvinylpyrrolidon,
- Mikroorganismen,
- Alginsäure-Derivate, wie beispielsweise Natriumalginat,
- Salze, wie beispielsweise Natriumcarbonat, Kaliumcarbonat, Calciumcarbonat, Ammoniumcarbonat, Magnesiumcarbonat, Natriumhydrogencarbonat, Kaliumhydrogencarbonat, Calciumhydrogencarbonat, Magnesiumhydrogencarbonat, Natriumsulfat, Kaliumsulfat, Ammoniumsulfat, Magnesiumsulfat, Natriumphosphat, Kaliumphosphat, Ammoniumphosphat, Magnesiumphosphat, Natriumchlorid, Kaliumchlorid, Ammoniumchlorid, Calciumchlorid, Magnesiumchlorid und/oder Aluminiumchlorid
verstanden.

Die erfindungsgemäßen Hydrogele zeichnen sich gegenüber Hydrogelen aus konventionellen Polymeren durch eine höherer Klarheit aus (Vergleich der Haze-Werte: Siehe Beispiele 3 und 4).

Die erfindungsgemäßen Hydrogele zeichnen sich gegenüber Hydrogelen aus konventionellen Polymeren durch eine höhere Beständigkeit gegenüber Wasser aus, was bei einem Einsatz als Trägermaterial für Einschluß - immobilisierte Biokatalysatoren, beispielsweise für die Abwasserbehandlung, von Vorteil ist (Vergleich der Beständigkeit gegenüber Wasser: Siehe Beispiele 7 und 8).

Erläuternde, die Erfindung jedoch nicht einschränkende Beispiele für die erfindungsgemäßen Hydrogele sind Produkte aus Mischungen von:
- Polyvinylalkohol-Sternpolymer(en), konventionellem Polyvinylalkohol und Wasser,
- Polyvinylalkohol-Sternpolymer(en), Polyacrylsäure und Wasser
- Polyvinylalkohol-Sternpolymer(en), Polymethacrylsäuren und Wasser
- Polyvinylalkohol-Sternpolymer(en), Polyacrylaten und Wasser
- Polyvinylalkohol-Sternpolymer(en), Polymethacrylaten und Wasser
- Polyvinylalkohol-Sternpolymer(en), Polyvinylpyrrolidon und Wasser
- Polyvinylalkohol-Sternpolymer(en), konventionellem Polyvinylalkohol, Natriumalginat, Calciumchlorid, Formaldehyd und Wasser,
- Polyvinylalkohol-Sternpolymer(en), konventionellem Polyvinylalkohol, Mikroorganismen und Wasser,
- Polyvinylalkohol-Sternpolymer(en), konventionellem Polyvinylalkohol, Calciumchlorid und Wasser,
- Polyvinylalkohol-Sternpolymer(en), konventionellem Polyvinylalkohol, Calciumchlorid, Mikroorganismen und Wasser.

Weiterer Gegenstand der Erfindung ist die Verwendung des Hydrogels enthaltend ein oder mehrere Polyvinylalkohol-Sternpolymer(e) sowie weitere Komponenten als:
- Materialien im biomedizinischen und pharmazeutischen Bereich, beispielsweise als künstliches Gewebe oder zur Wundbehandlung
- als Material zur Herstellung von Kontaktlinsen
- als Material zur kontrollierten Freisetzung von Wirkstoffen
- als Trägermaterial für Einschluß - immobilisierte Biokatalysatoren, beispielsweise für die Abwasserbehandlung
- als Trägermaterial für Übergangsmetallkatalysatoren, beispielsweise Palladiumkatalysatoren
- als Material für die Herstellung von Reaktivmembranen,
- als Zusatz zu Bohrflüssigkeiten, insbesondere, aufgrund der Abbaubarkeit, als Zusatz zu Bohrspülungen für Offshore-Applikationen
- als Verdrängungsmittel bei der Ölförderung
- als Additiv für Zement, insbesondere Dispersionen und Emulsionen
- als Zusatzmittel zu hochviskosen Flüssigkeiten, wie z.B. Erdöl zur Verbesserung des Fließverhaltens und zum schnelleren Transport
- als Bestandteil von Kosmetikartikeln.

Die Erfindung wird durch folgende, die Erfindung jedoch nicht einschränkende Beispiele erläutert.

### Beispiel 1: Herstellung eine Polyvinylalkohol-Sternpolymers gemäß DE10343607

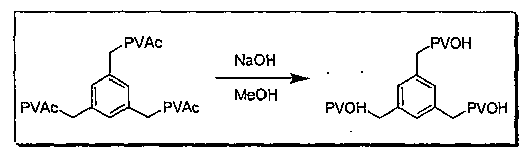

In einem 1000 ml Rundkolben werden 167 ml einer 1 %igen methanolischen Natronlauge im Wasserbad auf 50°C erwärmt. Hierzu wird über einen Zeitraum von 30 min. eine Lösung von 50 g Polyvinylacetat-Sternpolymer (hergestellt gemäß DE10238659) in 333 ml Methanol zugetropft. Nach Beendigung der Zugabe wird noch 30 min. gerührt. Der weiße Niederschlag wird abfiltriert, mit Methanol alkalifrei gewaschen, und im Vakuum getrocknet. Ausbeute: 25.0 g. ¹H-NMR (500 MHz, [D₆]-DSMO): δ = 6.65 (s, aromat. H), 4.65, 4.46, 3.89, 3.84, 3.31, 1.44 - 1.33 (4 x s, 1 x m, PVOH) ppm.

### Beispiel 2: Herstellung eine Polyvinylalkohol-Sternpolymers gemäß DE10356574

132 ml (1.43 mol) Vinylacetat, 9.0 g (70.2 mmol) Diallylformal, 1.6 ml (7.3 mmol) Tris-(2,2,2-trifluorethylphosphit und 1.25 g (3.6 mmol) Dibenzoylperoxid (70 % ig, Rest: H₂O) werden in 68 ml Toluol vorgelegt. Die klare farblose Lösung wird 20 h bei 70°C gerührt. Die flüchtigen Bestandteile werden im Ölpumpenvakuum entfernt und das Polyvinylacetat-Sternpolymer wird in 200 ml Methanol aufgenommen. Diese Lösung wird innerhalb von 15 min. zu 400 ml einer 1 %igen NaOH-Lösung in Methanol zugetropft. Es wird noch 90 min. bei 50°C gerührt. Der Polyvinylalkohol wird abfiltriert, mit Methanol neutral gewaschen und im Ölpumpenvakuum getrocknet. Ausbeute: 14.4 g (22 %) ¹H-NMR (500 MHz, [D₆]-DSMO): δ = 5.92 - 5.87 (m), 5.24, 5.13 (2 x d), 4.66, 4.61, 4.46, 4.22, 4.21, 4.10, 4.09, 4.00, 3.89, 3.84, 3.35, 3.16, 1.43 - 1.33 (13 x m) ppm.

### Beispiel 3: Herstellung eines Hydrogels enthaltend ein Polyvinylalkohol-Sternpolymere (Vergleichsbeispiel)

25 g Polyvinylalkohol-Sternpolymer (Beispiel 1) werden bei 70°C in 75 ml demineralisiertem Wasser gelöst. Die Lösung wird in eine flache Metallschale (20 x 20 cm) gegeben, mit einem Deckel versehen und 12 h bei -30°C aufbewahrt. Danach läßt man die Lösung auf innerhalb von 12 h auf Raumtemperatur kommen und wiederholt den Einfrier - Auftau - Vorgang noch zweimal. Das Hydrogel wird in Form eine 0.25 cm dicken Filmes erhalten. Trübung (Haze-Wert in Prozent): 1.3.

### Beispiel 4: Herstellung eines Hydrogels enthaltend einen linearen Polyvinylalkohol (Vergleichsbeispiel)

25 g Polyvinylalkohol (Celvol 103 der Fa. Celanese) werden bei 70°C in 75 ml demineralisiertem Wasser gelöst. Die Lösung wird in eine flache Metallschale (20 x 20 cm) gegeben, mit einem Deckel versehen und 12 h bei -30°C aufbewahrt. Danach läßt man die Lösung auf innerhalb von 12 h auf Raumtemperatur kommen und wiederholt den Einfrier - Auftau - Vorgang noch zweimal. Das Hydrogel wird in Form eine 0.25 cm dicken Filmes erhalten. Trübung (Haze-Wert in Prozent): 4.6.

### Beispiel 5: Herstellung eines Hydrogels enthaltend ein Gemisch eines Polyvinylalkohol-Sternpolymers mit einem linearen Polyvinylalkohol

2 g Polyvinylalkohol-Sternpolymer (Beispiel 2), 2 g Polyvinylalkohol (Celvol 103 der Fa. Celanese), 0.5 g Alginsäure-Natriumsalz und 0.15 g Natriumhydrogencarbonat werden bei 70°C in 50 ml Wasser gelöst und nach Abkühlen auf Raumtemperatur zu 250 ml einer 0.1 molaren Calciumchlorid-Lösung in großen Tropfen aus einer Spritze ohne Kanüle unter langsamem Rühren zugetropft, wobei sich ca. 3 mm große sphärische Partikel bilden. Die Partikel werden durch vorsichtige Filtration über eine Glasfritte isoliert und zu einer 40°C warmen Lösung von 5 g Formaldehyd (37 %ig in Wasser), 50 g konzentrierter Schwefelsäure und 25 g Natriumsulfat gegeben. Nach einer Stunde werden die verfestigten sphärischen Partikel durch Filtration isoliert und mit Wasser neutral gewaschen. Ausbeute: 43 g Hydrogel als sphärische Partikel.

### Beispiel 6: Herstellung eines Hydrogels enthaltend einen linearen Polyvinylalkohol (Vergleichsbeispiel)

4 g Polyvinylalkohol (Celvol 103 der Fa. Celanese), 0.5 g Alginsäure-Natriumsalz und 0.15 g Natriumhydrogencarbonat werden bei 70°C in 50 ml Wasser gelöst und nach Abkühlen auf Raumtemperatur zu 250 ml einer 0.1 molaren Calciumchlorid-Lösung in großen Tropfen aus einer Spritze ohne Kanüle unter langsamem Rühren zugetropft, wobei sich ca. 3 mm große sphärische Partikel bilden. Die Partikel werden.durch vorsichtige Filtration über eine Glasfritte isoliert und zu einer 40°C warmen Lösung von 5 g Formaldehyd (37 %ig in Wasser), 50 g konzentrierter Schwefelsäure und 25 g Natriumsulfat gegeben. Nach einer Stunde werden die verfestigten sphärischen Partikel durch Filtration isoliert und mit Wasser neutral gewaschen. Ausbeute: 37 g Hydrogel als sphärische Partikel.

### Beispiel 7: Untersuchung der Abnutzung des Hydrogels aus Beispiel 5

In eine 50 ml - Einwegspritze, auf deren Boden sich ein Plastiksieb (Maschenweite 0.75 mm) befindet, wurde 10 g Hydrogel aus Beispiel 5 gegeben. Das Hydrogel wurde mit einem Plastiksieb bedeckt und der Hohlraum über dem Hydrogel mit Glaswolle befüllt. Die Spritze wurde mit einem Gummistopfen, durch den ein 1 mm - Teflonschlauch führt, verschlossen. Mit Hilfe einer Pumpe wurde durch den Teflonschlauch über einen Zeitraum von einer Woche über das Hydrogel Leitungswasser mit einer Flußrate von 1 ml / min geleitet. Danach wurde das Hydrogel aus der Spritze entfernt und gewogen. Nach einer Woche wurden 9.2 g Hydrogel aus Beispiel 5 isoliert.

### Beispiel 8: Untersuchung der Abnutzung des Hydrogels aus Beispiel 6 (Vergleichsbeispiel)

In eine 50 ml - Einwegspritze, auf deren Boden sich ein Plastiksieb (Maschenweite 0.75 mm) befindet, wurde 10 g Hydrogel aus Beispiel 6 gegeben. Das Hydrogel wurde mit einem Plastiksieb bedeckt und der Hohlraum über dem Hydrogel mit Glaswolle befüllt. Die Spritze wurde mit einem Gummistopfen, durch den ein 1 mm - Teflonschlauch führt, verschlossen. Mit Hilfe einer Pumpe wurde durch den Teflonschlauch über einen Zeitraum von einer Woche über das Hydrogel Leitungswasser mit einer Flußrate von 1 ml / min geleitet. Danach wurde das Hydrogel aus der Spritze entfernt und gewogen. Nach einer Woche wurden 8.1 g Hydrogel aus Beispiel 6 isoliert.

## Patentansprüche

1. Hydrogele, enthaltend mindestens ein Polyvinylalkohol-Sternpolymer und weitere Komponenten.

2. Hydrogel gemäß Anspruch 1, **dadurch gekennzeichnet, dass** das Hydrogel neben dem Polyvinylalkohol-Sternpolymer weiterer Polyvinylalkohol-Sternpolymere enthält.

3. Hydrogel gemäß Anspruch 1, **dadurch gekennzeichnet, dass** das Hydrogel neben dem Polyvinylalkohol-Sternpolymer weiterer Polymere ausgewählt aus der Gruppe der Polyvinylalkohole, Polyacrylsäuren, Polymethacrylsäuren, Polyacrylaten, Polymethacrylaten und Polyvinylpyrrolidon enthält.

4. Hydrogel gemäß Anspruch 1, **dadurch gekennzeichnet, dass** das Polyvinylalkohol-Sternpolymer mindestens eine Verbindung der Formel **I**, **II** und **III** 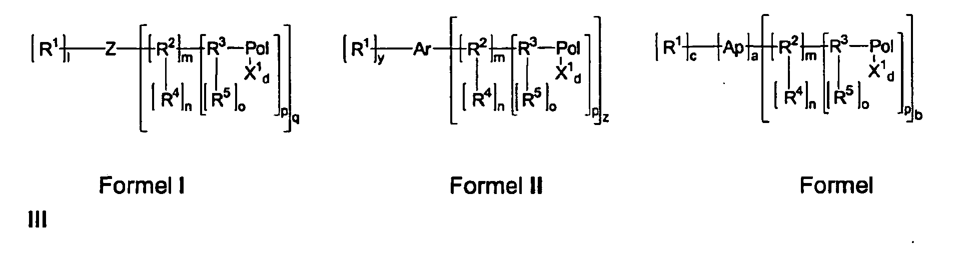 worin
Pol für ein Polymer auf der Basis eines Polyvinylalkohols steht, besonders be- vorzugt für ein Homo- oder Copolymer auf der Basis von Polyvinylalkohol, ganz besonders bevorzugt Polyvinylalkohol, Polyvinylalkohol- Polyvinylacetat-Copolymer, Polyvinylalkohol-Polyethylen-Copolymer, Pol- yvinylalkohol-Polyvinylchlorid-Copolymer und Polyvinylalkohol- Polyacrylsäuremethylester-Copolymer, und
Z ein Zentralatom bedeutet und ein Atom der 13. bis 16. Gruppe des Perio- densystems der Elemente, bevorzugt Kohlenstoff, Silicium, Stickstoff, Phosphor, Sauerstoff oder Schwefel, besonders bevorzugt Kohlenstoff oder Silicium, ist, und
X¹ jeweils gleich oder verschieden ist, und ein Halogenatom, bevorzugt Fluor, Chlor, Brom oder lod, besonders bevorzugt Chlor, Brom oder lod ist, und
R¹ gleich oder verschieden ist, und gleich Wasserstoff oder eine C₁ - C₂₀- kohlenstoffhaltige Gruppe ist, und
R² gleich oder verschieden ist und eine verbrückende C₁ - C₂₀ - kohlenstoffhaltige Gruppe zwischen dem Zentralatom Z und der initiieren- den Einheit [R³-X¹] oder Silicium oder Sauerstoff bedeutet, und
R³ gleich oder verschieden ist und Kohlenstoff oder Silicium bedeutet, und
R⁴ gleich oder verschieden ist und ein Wasserstoffatom oder eine C₁ - C₂₀ - kohlenstoffhaltige Gruppe ist, und
R⁵ gleich oder verschieden ist und Wasserstoff oder eine C₁ - C₂₀ - kohlen- stoffhaltige Gruppe bedeutet,
l eine ganze natürliche Zahl ist und für Null, 1, 2 oder 3 steht, und
m jeweils gleich oder verschieden ist und eine ganze natürlich Zahl ist und für Null, 1, 2, 3, 4 und 5 steht, und
n jeweils gleich oder verschieden ist und eine ganze natürliche Zahl ist und für Null, 1, 2, 3, 4, 5, 6, 7, 8, 9, 10, 11, 12, 13, 14, 15, 16, 17, 18, 19 und 20 steht, und
o jeweils gleich oder verschieden ist und 1 oder 2 ist, und
p jeweils gleich oder verschieden ist und eine ganze natürliche Zahl ist und für 1, 2, 3, 4 und 5 steht, und
q eine ganze natürliche Zahl ist und für 2, 3 und 4 steht, und
Ar ein aromatisches Grundgerüst mit mindestens vier Kohlenstoffatomen ist, bei dem ein oder mehrere C-Atome durch Bor, Stickstoff oder Phosphor ausgetauscht sein können, und wobei bevorzugte aromatische oder hete- roaromatische Grundgerüste sich von Benzol, Biphenyl, Naphthalin, Anthracen, Phenanthren, Triphenylen, Chinolin, Pyridin, Bipyridin, Pyrida- zin, Pyrimidin, Pyrazin, Triazin, Benzopyrrol, Benzotriazol, Benzopyridin, Benzopyrazidin, Benzopyrimidin, Benzopyrazin, Benzotriazin, Indolizin, Chinolizin, Carbazol, Acridin, Phenazin, Benzochinolin, Phenoxazin, die gegebenenfalls auch substituiert sein können, ableiten, und
y eine ganze natürliche Zahl ist und für Null, 1, 2, 3, 4, 5, 6, 7, 8, 9, 10, 11, 12, 13, 14, 15, 16, 17, 18, 19 und 20 steht, und
z eine ganze natürliche Zahl ist und für 2, 3, 4, 5, 6, 7, 8, 9 und 10 steht.
Ap ein cyclisches nicht-aromatisches Grundgerüst mit mindestens drei Kohlen- stoffatomen ist, welches auch Heteroatome wie Stickstoff, Bor, Phosphor, Sauerstoff oder Schwefel enthalten kann, ist, wobei sich bevorzugte alipha- tische Grundgerüste aus der Gruppe Cycloalkyl, wie beispielsweise Cyc- lopropyl, Cyclobutyl, Cyclopentyl, Cyclohexyl, Cycloheptyl, Cyclooctyl, Cyc- lononyl, oder aus der Gruppe Cycloheteroalkyl, wie beispielsweise Aziridin, Azetidin, Pyrrolidin, Piperidin, Azepan, Azocan, 1,3,5-Triazinan, 1,3,5- Trioxan, Oxetan, Furan, Dihydrofuran, Tetrahydrofuran, Pyran, Dihydropy- ran, Tetrahydropyran, Oxepan, Oxocan, oder aus der Gruppe der Saccha- ride, wie beispielsweise alpha-Glucose, beta-Glucose, ableiten lassen, und
a eine ganze natürliche Zahl ist und für Null, 1, 2, 3, 4, 5, 6, 7, 8, 9, 10, 11, 12, 13, 14, 15, 16, 17, 18, 19 und 20 steht, und
b eine ganze natürliche Zahl ist und für 2, 3, 4, 5, 6, 7, 8, 9 und 10 steht, und
c eine ganze natürliche Zahl ist und für Null, 1, 2, 3, 4, 5, 6, 7, 8, 9, 10, 11, 12, 13, 14, 15, 16, 17, 18, 19 und 20 steht,
d gleich oder verschieden sein kann, und Null oder Eins ist, ist.

5. Hydrogel gemäß Anspruch 1, **dadurch gekennzeichnet, dass** das Polyvinylalkohol-Sternpolymer ein Polyvinylalkohol-Copolymer auf der Basis von Polyvinylester-Copolymeren ist, die durch ein Verfahren umfassend die Schritte:
A) Radikalische Lösungs- oder Massen-Polymerisation von Vinylestern in Gegenwart eines Radikalbildners und gegebenenfalls in Gegenwart eines Radikalfängers,
B) Zugabe einer zur Vernetzung befähigten Polyalkenyl-Verbindung,
C) gegebenenfalls Aufarbeitung und Isolierung der gebildeten Polyvinylester-Copolymere,
D) Verseifung der in B) hergestellten Polyvinylester/Polyvinylesterpolyalken-Mischung oder der unter C) isolierten Polyvinylesterpolyalken-Copolymere mit Base unter Ausbildung der Polyvinylalkohol)/Polyvinylalkohol-polyalken-Mischungen bzw. Polyvinylalkoholpolyalken-Copolymeren und Isolierung der Produkte,
erhältlich sind,
**dadurch gekennzeichnet, dass** es sich bei den in Schritt B) eingesetzten Polyalkenyl-Verbindungen um Verbindungen gemäß Formel **IV:** 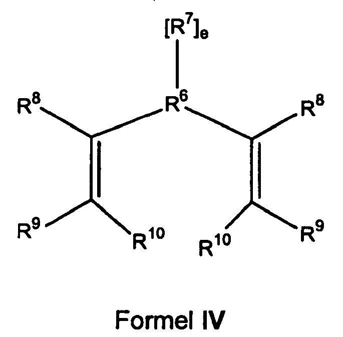 worin:
R⁶ eine C₆ - C₂₀ -Arylgruppe, eine C₅ - C₂₀ -Heteroarylgruppe, eine C₄-C₂₀- Cycloalkylgruppe, eine C₄-C₂₀-Heterocycloalkylgruppe oder eine C₁ - C₂₀- Alkylgruppe bedeutet, bei der ein oder mehrere nicht direkt benachbarte C- Atome durch ein Element der 5. oder 6. Gruppe der Elemente, bevorzugt Stickstoff, Phosphor, Sauerstoff oder Schwefel, besonders bevorzugt Stick- stoff oder Sauerstoff, ersetzt sein können, und
R⁷ gleich oder verschieden ist, und gleich Wasserstoff, Sauerstoff, Schwefel oder eine Hydroxygruppe, eine Carbamoylgruppe, eine Aminogruppe, eine Carboxygruppe, eine C₁ - C₂₀ -Alkylcarbonylgruppe, eine C₁ - C₂₀ - Alkyloxygruppe, eine C₆ - C₂₀ -Aryloxygruppe, eine Iminogruppe, eine C₁ - C₂₀-Alkyliminogruppe, eine C₆ - C₂₀ -Alkyliminogruppe, eine Cyanogruppe, eine C₁ - C₂₀- Alkylgruppe, eine C₆ - C₂₀ -Arylgruppe, eine C₅ - C₂₀- Heteroarylgruppe, eine C₄-C₂₀-Cycloalkylgruppe, eine C₄-C₂₀- Heterocycloalkyl eine C₇ - C₂₀ -Alkylarylgruppe, eine C₇ - C₃₀- Arylalkylgruppe, eine C₂ - C₂₀ -Alkenylgruppe, eine C₂ - C₂₀ - α-Oxy- alkenyl, eine halogenhaltige C₁ - C₂₀ -Alkylgruppe, eine C₆ - C₂₀- Arylgruppe, eine C₇ - C₂₀ -Alkylarylgruppe, eine C₇ - C₃₀ -Arylalkylgruppe oder eine C₂ - C₂₀ -Alkenylgruppe bedeutet, und
R⁸, R⁹, R¹⁰ gleich oder verschieden sind, und gleich Wasserstoff oder eine C₁ - C₂₀ - kohlenstoffhaltige Gruppe ist, und
e eine ganze natürliche Zahl von 0 bis 40 bedeutet, handet.

6. Verwendung von Hydrogelen, enthaltend mindestens ein Polyvinylalkohol-Sternpolymer, im biomedizinischen und pharmazeutischen Bereich, insbesondere zur Herstellung von künstlichem Gewebe oder zur Wundbehandlung, zur Herstellung von Kontaktlinsen, als Trägermaterial zur kontrollierten Freisetzung von Wirkstoffen, als Trägermaterial für Einschluß - immobilisierte Biokatalysatoren, insbesondere in der Abwasserbehandlung, als Trägermaterial für Übergangsmetallkatalysatoren, als Material für die Herstellung von Reaktivmembranen, als Zusatz zu Bohrflüssigkeiten, insbesondere in Bohrspülungen, als Verdrängungsmittel bei der Ölförderung, als Additiv in Baustoffen, insbesondere Dispersionen und Emulsionen, als Zusatzmittel zu hochviskosen Flüssigkeiten, insbesondere zur Verbesserung des Fließverhaltens und zum schnelleren Transport, und als Bestandteil von Kosmetikartikeln.

## Claims

1. Hydrogels containing at least one polyvinyl alcohol star polymer and further components.

2. The hydrogel according to Claim 1, **characterized in that** the hydrogel contains, in addition to the polyvinyl alcohol star polymer, further polyvinyl alcohol star polymers.

3. The hydrogel according to Claim 1, **characterized in that** the hydrogel contains, in addition to the polyvinyl alcohol star polymer, further polymers selected from the group consisting of polyvinyl alcohols, polyacrylic acids, polymethacrylic acids, polyacrylates, polymethacrylates and polyvinylpyrrolidone.

4. The hydrogel according to Claim 1, **characterized in that** the polyvinyl alcohol star polymer is at least one compound of formula **I, II** or **III** 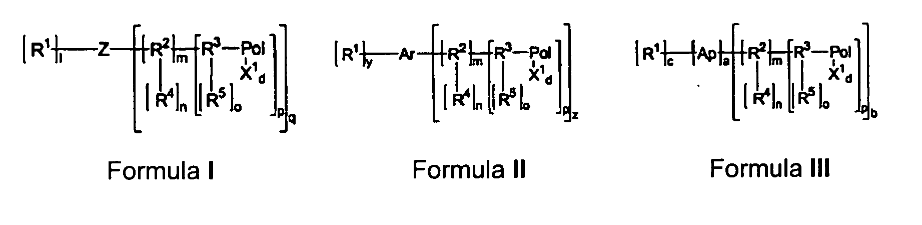 in which
Pol represents a polymer based on a polyvinyl alcohol, particularly preferably a homopolymer or copolymer based on polyvinyl alcohol, very particularly preferably polyvinyl alcohol, polyvinyl alcohol/polyvinyl acetate copolymer, polyvinyl alcohol/polyethylene copolymer, polyvinyl alcohol/polyvinyl chloride copolymer or polyvinyl alcohol/polyacrylic acid methyl ester copolymer, and
Z represents a central atom and is an atom from the 13th to 16th group of the periodic table of the elements, preferably carbon, silicon, nitrogen, phosphorus, oxygen or sulphur, particularly preferably carbon or silicon, and
X¹ is in each case identical or different and is a halogen atom, preferably fluorine, chlorine, bromine or iodine, particularly preferably chlorine, bromine or iodine, and
R¹ is identical or different and is hydrogen or a C₁-C₂₀ carbon-containing group, and
R² is identical or different and is a bridging C₁-C₂₀ carbon-containing group between the central atom Z and the initiating unit [R³-X¹] or is silicon or oxygen,and
R³ is identical or different and is carbon or silicon, and
R⁴ is identical or different and is a hydrogen atom or a C₁-C₂₀ carbon-containing group, and
R⁵ is identical or different and is hydrogen or a C₁-C₂₀ carbon-containing group,
I is a natural integer and is zero, 1, 2 or 3, and
m is in each case identical or different and is a natural integer and is zero, 1, 2, 3, 4 or 5, and
n is in each case identical or different and is a natural integer and is zero, 1, 2, 3, 4, 5, 6, 7, 8, 9, 10, 11, 12, 13, 14, 15, 16, 17, 18, 19 or 20, and
o is in each case identical or different and is 1 or 2, and
p is in each case identical or different and is a natural integer and is 1, 2, 3, 4 or 5, and
q is a natural integer and is 2, 3 or 4, and
Ar is an aromatic skeleton having at least four carbon atoms, in which one or more C atoms may be replaced by boron, nitrogen or phosphorus, and wherein preferred aromatic or heteroaromatic skeletons derive from benzene, biphenyl, naphthalene, anthracene, phenanthrene, triphenylene, quinoline, pyridine, bipyridine, pyridazine, pyrimidine, pyrazine, triazine, benzopyrrole, benzotriazole, benzopyridine, benzopyrazidine, benzopyrimidine, benzopyrazine, benzotriazine, indolizine, quinoiizine, carbazole, acridine, phenazine, benzoquinoline, phenoxazine, which may optionally also be substituted, and
y is a natural integer and is zero, 1, 2, 3, 4, 5, 6, 7, 8, 9, 10, 11, 12, 13, 14, 15, 16, 17, 18, 19 or 20, and
z is a natural integer and is 2, 3, 4, 5, 6, 7, 8, 9 or 10,
Ap is a cyclic, non-aromatic skeleton having at least three carbon atoms, which may also contain heteroatoms such as nitrogen, boron, phosphorus, oxygen or sulphur, wherein preferred aliphatic skeletons may derive from the cycloalkyl group, such as for example cyclopropyl, cyclobutyl, cyclopentyl, cyclohexyl, cycloheptyl, cyclooctyl, cyclononyl, or from the cycloheteroalkyl group, such as for example aziridine, azetidine, pyrrolidine, piperidine, azepane, azocane, 1,3,5-triazinane, 1,3,5-trioxane, oxetane, furan, dihydrofuran, tetrahydrofuran, pyran, dihydropyran, tetrahydropyran, oxepane, oxocane, or from the saccharide group, such as for example alpha- glucose, beta-glucose, and
a is a natural integer and is zero, 1, 2, 3, 4, 5, 6, 7, 8, 9, 10, 11, 12, 13, 14, 15, 16, 17, 18, 19 or 20, and
b is a natural integer and is 2, 3, 4, 5, 6, 7, 8, 9 or 10, and
c is a natural integer and is zero, 1, 2, 3, 4, 5, 6, 7, 8, 9, 10, 11, 12, 13, 14, 15, 16, 17, 18, 19 or 20,
d may be identical or different and is zero or one.

5. The hydrogel according to Claim 1, **characterized in that** the polyvinyl alcohol star polymer is a polyvinyl alcohol copolymer based on polyvinyl ester copolymers, which are obtainable by a method comprising the steps:
A) radical solution or mass polymerization of vinyl esters in the presence of a radical generator and optionally in the presence of a radical scavenger,
B) addition of a polyalkenyl compound capable of crosslinking,
C) optionally, work-up and isolation of the polyvinyl ester copolymers formed,
D) saponification of the polyvinyl ester/polyvinyl ester polyalkene mixture produced in B) or of the polyvinyl ester/polyalkene copolymers isolated in C) with a base to form the polyvinyl alcohol/polyvinyl alcohol polyalkene mixtures or polyvinyl alcohol/polyalkene copolymers and isolation of the products,
**characterized in that** the polyalkenyl compounds used in step B) are compounds according to Formula IV: 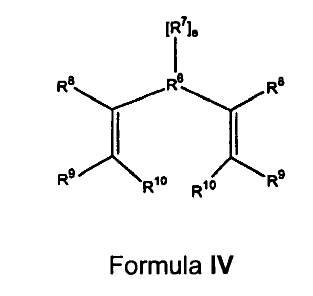 in which:
R⁶ is a C₆-C₂₀ aryl group, a C₅-C₂₀ heteroaryl group, a C₄-C₂₀ cycloalkyl group, a C₄-C₂₀ heterocycloalkyl group or a C₁-C₂₀ alkyl group, in which one or more C atoms which are not directly adjacent to one another may be replaced by an element of the 5th or 6th group of the elements, preferably nitrogen, phosphorus, oxygen or sulphur, particularly preferably nitrogen or oxygen, and
R⁷ is identical or different and is hydrogen, oxygen, sulphur or a hydroxyl group, a carbamoyl group, an amino group, a carboxy group, a C₁-C₂₀ alkylcarbonyl group, a C₁-C₂₀ alkyloxy group, a C₆-C₂₀ aryloxy group, an imino group, a C₁-C₂₀ alkylimino group, a C₆-C₂₀ alkylimino group, a cyano group, a C₁-C₂₀ alkyl group, a C₆-C₂₀ aryl group, a C₅-C₂₀ heteroaryl group, a C₄-C₂₀ cycloalkyl group, a C₄-C₂₀ heterocycloalkyl, a C₇-C₂₀ alkylaryl group, a C₇-C₃₀ arylalkyl group, a C₂-C₂₀ alkenyl group, a C₂-C₂₀ α-oxyalkenyl, a halogen- containing C₁-C₂₀ alkyl group, a C₆-C₂₀ aryl group, a C₇-C₂₀ alkylaryl group, a C₇-C₃₀ arylalkyl group or a C₂-C₂₀ alkenyl group, and
R⁸, R⁹, R¹⁰ are identical or different and are hydrogen or a C₁-C₂₀ carbon- containing group, and
e is a natural integer from 0 to 40.

6. The use of hydrogels containing at least a polyvinyl alcohol star polymer in the biomedical and pharmaceutical field, in particular for producing artificial tissue or for treating wounds, for producing contact lenses, as a carrier material for the controlled release of active substances, as a carrier material for inclusion-immobilized biocatalysts, in particular in wastewater treatment, as a carrier material for transition metal catalysts, as a material for producing reactive membranes, as an additive to drilling fluids, in particular in flushing fluids, as a displacing agent in oil recovery, as an additive in building materials, in particular dispersions and emulsions, as an additive to highly viscous fluids, in particular to improve the flowability and increase the speed of conveyance, and as a component of cosmetic products.

## Revendications

1. Les hydrogels contenant au moins un polymère d'alcool de polyvinyle en étoile et d'autres composantes.

2. Un hydrogel selon revendication 1, **caractérisé en ce que** l'hydrogel contient, en plus du polymère en étoile alcool de polyvinyle, d'autres polyvinyles en étoile polymères d'alcool.

3. Un hydrogel selon revendication 1, **caractérisé en ce que** l'hydrogel contient, en plus du polymère en étoile alcool de polyvinyle, d'autres polymères, choisis dans le groupe des alcools de polyvinyles, des acides polyacryliques, des acides poly-méthacryliques, des polyacrylates, des poly-méthacrylates et des pyrroliques de polyvinyle.

4. Un hydrogel selon revendication 1, **caractérisé en ce que** le polymère en étoile polyvinyle d'alcool contient au moins une relation conforme aux formules I, II et III 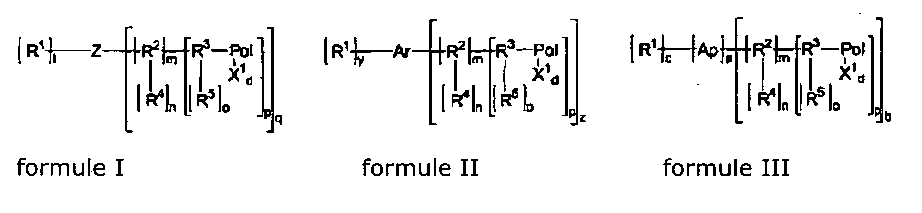 dans lesquelles:
Pol est un polymère sur la base d'un alcool polyvinylique, particulièrement préconisé pour homo- polymère ou un copolymère sur la base d'alcool de polyvinyle, particulièrement préconisés les alcools polyvinyliques, les alcools de polyvinyle - acétates de polyvinyle - copolymères, les alcool polyvinyliques - les polyéthylènes - les copolymères, les alcool polyvinyliques - les chlorites polyvinyliques - les copolymères et les alcool polyvinyliques - les esters méthyliques d'acides polyacryliques et
Z Un atome central et un atome du 13ème au 16ème groupe du système périodique des éléments; le carbone, le silicium, l'azote, le phosphore, l'oxygène ou le souffre seront préconisés, et le carbone ou le silicium seront particulièrement préconisés, et
X¹ sera identique ou différent, et représente un atome halogène, préconisés seront le fluor, le chlore, le brome ou l'iode, et le chlore, le brome ou l'iode particulièrement préconisés, et
R¹ sera identique ou différent, et sera de l'hydrogène ou un groupe C₁-C₂₀ riche en carbone, et
R² sera identique ou différent, et un groupe liant C₁-C₂₀ riche en carbone entre l'atome central Z et l'unité d'initiation [R³-X¹] ou sera du silicium ou de l'oxygène, et
R³ sera identique ou différent et sera du carbone ou du silicium, et
R⁴ sera identique ou différent et représente un atome d'hydrogène ou un groupe C₁-C₂₀ riche en carbone, et
R⁵ sera identique ou différent et représente de l'hydrogène ou un groupe C₁-C₂₀ riche en carbone, et
l est un nombre entier naturel et représente nul, 1 ,2 ou 3, et
m sera identique ou différent et représente un nombre entier naturel nul, 1, 2, 3, 4, et 5, et
n sera identique ou différent et représente un nombre entier naturel nul, 1, 2, 3, 4, 5, 6, 7, 8, 9, 10, 11, 12, 13, 14, 15, 16, 17, 18, 19 et 20, et
o sera identique ou différent, et sera égal à 1 ou 2, et
p sera identique ou différent et représente un nombre entier naturel nul, 1, 2, 3, 4, et 5, et
q est un nombre entier naturel et représente 2, 3 et 4, et
Ar est un édifice structural aromatique comprenant au moins quatre atomes de carbone, dans laquelle un ou plusieurs atomes de C peuvent être remplacés par du bore, de l'azote ou du phosphore et dans la quelle les bases aromatiques ou hétéro aromatiques préconisées seront dérivées du benzène, du bi phényle, de la naphtaline, des anthracènes, des phénanthrènes, des triphénylènes, de la chinoline, de pyridine, de bipyridine, de pyridazine, de pyrimidine, de pyrazine, de triazine, de pyroles de benzène, de triazole de benzène, de pyridine de benzène, de pyradizine de benzène, de pyrimidine de benzène, de pyrazine de benzène, de triazine de benzène, d'indolisine, de chinoline, de carbazole, d'acridine, de phénazine, de chinoline de benzène, de phenoxazine, qui pourront éventuellement être remplacés par des substituts, et y représente un nombre entier naturel nul, 1, 2, 3, 4, 5, 6, 7, 8, 9, 10, 11, 12, 13, 14, 15, 16, 17, 18, 19 et 20, et
z représente un nombre entier 2, 3, 4, 5, 6, 7, 8, 9, 10, et
Ap est un édifice structural cyclique et non aromatique comprenant au moins trois atomes de carbone, qui peuvent contenir des hétéro atomes comme de l'azote, du bore, du phosphore, de l'oxygène ou du souffre, de laquelle peuvent se laisser dériver des liaisons aliphatiques préférentielles des groupes des cycloalcènes, comme par exemple le cyclopropyle, le cycobutile, le cyclopentyle, le cyclohexile, le cycloheptyle, le cyclooctyile, le cyclononyl, ou du groupe des cycloheteroalkyles, comme par exemple l'aziridine, l'azetidine, la pyrrolidine, la piperidine, l'azepan, l'azocane, le triazinane 1, 2, et 5, la trioxane 1 ; 3 ; et 5, l'oxétane, la furane, la dihydrofurane, la tétrahydrofurane, la pyrane, la dihydropyrane, la tétrahydropyrane, l'oxépane, l'oxocane, ou du groupe des saccharides comme par exemple l'alpha glucose, le béta glucose, et
a représente un nombre entier naturel nul, 1, 2, 3, 4, 5, 6, 7, 8, 9, 10, 11, 12, 13, 14, 15, 16, 17, 18, 19 et 20, et
b représente un nombre entier naturel 2, 3, 4, 5, 6, 7, 8, 9 et 10 et
c représente un nombre entier naturel nul, 1, 2, 3, 4, 5, 6, 7, 8, 9, 10, 11, 12, 13, 14, 15, 16, 17, 18, 19 et 20, et
d pouvant être identique ou différent et égal à zéro ou à un.

5. Un hydrogel selon revendication 1, **caractérisé en ce que** le polymère en étoile polyvinyle d'alcool est un alcool de polyvinyle-copolymère sur la base d'ester de polyvinyle-copolymère que l'on peut obtenir avec le procédé suivant:
A) Polymérisation de solutions ou de masse radicales d'ester de vinyle en présence d'un durcisseur radical et éventuellement en présence d'un coagulant radical,
B) Rajout d'une liaison polyalkinique pouvant créer des réseaux,
C) Traitement éventuel et isolation de la copolymère d'ester de polyvinyle ainsi formée.
D) Hydrolisation du mélange comme décrit sous B) ester polyvinylique-ester polyvinylique-poly alcalidique-copolymères avec la formation d'un mélange polyvinyle d'alcool / polyvinyle d'alcool -polyalcalique ou/et polyvinyle d'alcool - polyalcalique- copolymères et isolation des produits, **caractérisé en ce que** due à la liaison utilisée sous C) est une liaison polyalcalique selon la formule IV 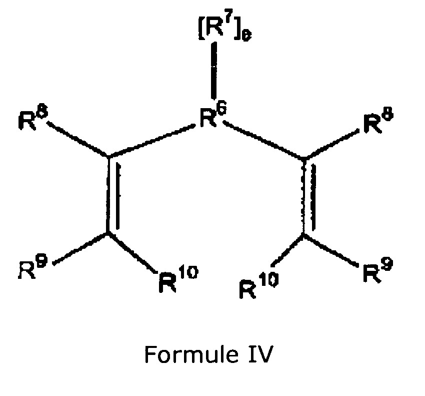 où :
R⁶ représente un groupe de type aryl C₆-C₂₀, un groupe de type hétéroaryl C₅ -C₂₀, un groupe de type cycloalkyl C₄-C₂₀, un groupe de type hétérocycloalkyl C₄-C₂₀ ou un groupe de type alkyl C₁-C₂₀, dans lequel un ou plusieurs atomes de C, non directement voisins, peuvent être remplacés par un élément du 5ème ou du 6ème groupe des éléments, avec une préconisation pour l'azote, le phosphore, l'oxygène, ou le soufre, sont particulièrement préconisés, l'azote et l'oxygène et
R⁷ est identique ou différent, et représente de l'hydrogène, de l'oxygène, du soufre, ou un groupe hydroxyque, un groupe carbonyl, un groupe amino, un groupe carboxy, un groupe de type C₁-C₂₀ Alkylcarbolil, un groupe de type C₁-C₂₀ alkyloxy, un groupe de type C₆-C₂₀ aryloxy, un groupe imino, un groupe de type C₁-C₂₀ alkylimino, un groupe de type C₆-C₂₀ alkylimino, un groupe cyano, un groupe de type C₁-C₂₀ alkyl, un groupe de type C₆-C₂₀ Aryl , un groupe de type C₅-C₂₀ hétéroaryl, un groupe de type C₄-C₂₀ cycloalkyl, un groupe de type C₇-C₃₀ aryalkyl, un groupe de type C₂-C₂₀ alkenyl, un groupe de type C₂-C₂₀ alpha-Oxyalkenyl, un groupe de type C₁-C₂₀ contenant des halogènes, un groupe de type C₇-C₂₀ Aryl, , un groupe de type C₇-C₂₀ alkylaryl, un groupe de type C₇-C₃₀ Arylalkyl ou , un groupe de type C₂-C₂₀ Alkenyl, et
R⁸, R⁹, R¹⁰ sont identiques ou différents et sont de l'oxygène ou un groupe de type C₁-C₂₀ contenant du carbone, ou
e est un nombre entier naturel compris entre 0 et 40,

6. Les hydrogels contenant au moins un polymère de base alcool de polyvinyle sont utilisés dans le secteur biomédical et pharmaceutique, en particulier à la fabrication de tissus artificiel ou pour soigner des plaies, à la fabrication de lentilles de contact, comme substrat lors de libérations contrôlées des agents actifs, comme substrats pour inclusions - biocatalyseurs immobilisés, en particulier dans le traitement des eaux usées, comme substrat pour des catalyseurs de liaisons métalliques, comme matériaux pour la fabrication des membranes réactives, comme additif à des liquides de perçage, en particulier pour le rinçage des perçages, comme moyen de refoulement pour l'exploitation pétrolière, comme additif dans les matériaux de construction, en particulier pour favoriser les dispersions et les émulsions, comme additif à des liquides à haute viscosité, en particulier pour l'amélioration de la fluidité et pour un écoulement plus rapide et comme composante de produits cosmétiques.
